# EUROPEAN PATENT APPLICATION

(11) **EP 3 413 036 A1**
(43) Date of publication of application: **12.12.2018**
(21) Application number: 17866381.1
(22) Date of filing: 14.11.2017
(51) Int. Cl.: G01N 21/81

(54) **ONLINE MONITORING AND REGENERATION SYSTEM FOR POWER TRANSFORMER BREATHER**

(30) Priority: 25.04.2017 CN 201710279635
(71) Applicant: Shandong Power Equipment Company. Ltd., Shandong 250022 (CN); State Grid Shandong Electric Power Company Jinan Power Supply Company, Shizhong District Jinan Shandong 250012 (CN)
(72) Inventor: TAN, Chong, Jinan, Shandong 250022 (CN); LIU, Ziting, Jinan, Shandong 250022 (CN); XU, Tao, Jinan, Shandong 250022 (CN); WANG, Xingang, Jinan, Shandong 250022 (CN); WANG, Yonggang, Jinan, Shandong 250022 (CN); JIANG, Lianggang, Jinan, Shandong 250022 (CN); LIU, Hengzhi, Jinan, Shandong 250022 (CN); REN, Bin, Jinan, Shandong 250022 (CN)
(74) Representative: EP&C
(86) International application number: PCT/CN2017/110960
(87) International publication number: WO 2018/196340

(57) **Abstract**

The disclosure provides an online monitoring and regeneration system for a power transformer respirator comprising: a power transformer respirator, a data collection terminal, a cloud server terminal and a monitoring terminal. The power transformer respirator is connected to the data collection terminal, the data collection terminal is connected to the cloud server terminal through a wireless network, and the cloud server terminal is connected to the monitoring terminal through a wireless network; the data collection terminal is configured to collect operating status data of the power transformer respirator and image information including allochroic silicagel of the power transformer respirator, and send the operating status data and the image information to the cloud server terminal; the cloud server terminal is configured to store the operating status data and the image information; and the monitoring terminal is configured to communicate with the cloud server terminal, and obtain and output the operating status data and the image information.

## Description

### CROSS-REFERENCE TO RELATED APPLICATIONS

The present application is based on and claims benefit of Chinese Patent Application No. 201710279635.3, filed on April 25, 2017, the contents of which are hereby incorporated by reference in its entirety.

### TECHNICAL FIELD

The disclosure relates to the field of power transformer respirator, and particularly to an online monitoring and regeneration system for power transformer respirator.

### BACKGROUND

A power transformer respirator is a device for removing and drying impurities and moisture entering the transformer and an oil conservator due to a change in transformer oil temperature, to ensure insulating strength of the transformer oil. A chemically treated drying agent, i.e., allochroic silicagel, is contained in a conventional power transformer respirator. The allochroic silicagel silica gel is usually blue under a dry condition, the allochroic silicagel gradually turns into pink after absorbing moisture and the silicagel of which a color changes gets ineffective. The operation and maintenance staff must constantly inspect, and timely replace the allochroic silicagel once its color changes. This not only increases a workload but also increases security risks for the normal operation of the power transformer. Moreover, there is a high cost for replacing of the allochroic silicagel, and the replaced allochroic silicagel may also bring the problems of environmental pollution and waste disposal.

With gradual popularization of construction and intelligent operation and maintenance of intelligent substations, the power transformer respirator is required to have functions of monitoring, control, maintenance-free and the like to further improve an intelligence level of a power transformer. However, the conventional power transformer respirator can no longer meet actual needs.

### SUMMARY

In view of the deficiencies of the existing background technology, embodiments of the disclosure provide an online monitoring and regeneration system for a power transformer respirator.

The online monitoring and regeneration system for power transformer respirator according to the embodiments of the disclosure includes: a power transformer respirator, a data collection terminal, a cloud server terminal and a monitoring terminal. The power transformer respirator is connected to the data collection terminal. The data collection terminal and the cloud server terminal are connected through a wireless network, and the cloud server terminal and the monitoring terminal are connected through the wireless network.

The data collection terminal is configured to obtain operating status data of the power transformer respirator and image information including allochroic silicagel of the power transformer respirator, and send the operating status data and the image information to the cloud server terminal.

The cloud server terminal is configured to store the operating status data and the image information.

The monitoring terminal is configured to communicate with the cloud server terminal, and obtain and output the operating status data and the image information.

In one embodiment, the data collection terminal includes:
a respirator status data collection module configured to obtain the operating status data of the power transformer respirator;
an image information collection module configured to obtain the image information including the silicagel of the power transformer respirator;
an embedded central processing module configured to process the respirator status data and the image information; and
a power management module configured to supply power to the respirator status data collection module, the image information collection module and the embedded central processing module.

In one embodiment, the power transformer respirator includes a mounting flange, a moisture-absorbing barrel with the allochroic silicagel and a metal thin film filter. The mounting flange connects an air tube of a transformer oil conservator and an air outlet of the power transformer respirator. The mounting flange is connected to the moisture-absorbing barrel through a connecting flange, the moisture-absorbing barrel is connected to the metal thin film filter through bolts. The bottom edge of the moisture-absorbing barrel is connected to the metal thin film filter with an O-type sealing ring. In one embodiment, the respirator status data collection module includes a temperature sensor, a humidity sensor and a pressure sensor which are located at a connection of the mounting flange and the moisture-absorbing barrel and are connected with the embedded central processing module through a communication cable.

The image information collection module includes a camera for collecting a color image of the allochroic silicagel in the moisture-absorbing barrel. The image information collection module is connected to the embedded central processing module through the wireless network, and transmits the image data collected by the camera to the embedded central processing module in the form of data flow.

The embedded central processing module is connected to a housing of the moisture-absorbing barrel through bolts.

In one embodiment, the embedded central processing module includes a processor and the following modules connected to the processor: a data storage module, a human-computer interaction system, a data transmission module, a clock module, an alarm module and a heating control module.

The data storage module is configured to store the respirator status data and the image information;
The human-computer interaction system includes a display component and an input component.

The data transmission module includes a wireless communication module and a data transmission interface, and the data transmission interface includes at least one of the following interface types: an RS485 interface, a Universal Serial Bus interface, abbreviated as USB interface, or an RJ45 interface.

The clock module uses a built-in Real Time Clock, abbreviated as RTC, and the clock module further includes a power supply configured to supply power to the RTC.

The alarm module is configured to give an alarm when the processor determines that the power transformer respirator fails based on the obtained operating status data of the power transformer respirator and image information, and the alarm module includes a buzzer and/or an indicator lamp.

In one embodiment, the heating control module includes an allochroic silicagel heater, a metal thin film filter heater and a relay.

The allochroic silicagel heater is located in the moisture-absorbing barrel, and the metal thin film filter heater is located in the metal thin film filter and close to a metal filter screen in the filter.

The allochroic silicagel heater and the metal thin film filter heater are both be connected to the embedded central processing module through a cable, the relay is located on a circuit board of the embedded central processing module, and startup or shutdown of the two heaters are respectively controlled by the pull-in or pull-out of contacts of the relay

In one embodiment, the power management module includes an Alternating Current power component, abbreviated as AC power component, a photovoltaic power component and a storage battery.

When the power management module includes the AC power component, the power management module further includes an AC adapter, and the AC power component is connected to the AC adapter.

In one embodiment, the wireless communication module includes a General Packet Radio Service module, abbreviated as GPRS module.

The cloud server terminal includes:
a cloud server configured to identify and accept connection requests sent by the data collection terminal and perform service responses to receive, parse, and store the data;
a network client configured to monitor operating status of the power transformer respirator in real time after a user logs in, and set status parameters to control regeneration of the allochroic silicagel of the power transformer respirator; and
a database configured to store and manage the operating status parameters of the power transformer respirator and user information.

In one embodiment, types of the monitoring terminal include a Personal Computer, abbreviated as PC, a tablet computer and a mobile phone.

The online monitoring and regeneration system for a power transformer respirator according to the embodiments of the disclosure, on the one hand, applies embedded technology, optical fiber sensing technology, image collection technology, wireless communication technology and cloud server technology to status data collection, storage, transmission and remote monitoring of the power transformer respirator to realize online monitoring and regeneration of the power transformer respirator. On the other hand, the shutdown time of the transformer can be reduced, and the reliability of power supply reliability can be improved. In terms of safe operation, insulation performance of the transformer can be improved, and safe operation of equipment can be ensured. In terms of economical operation, a replacement frequency of the allochroic silicagel can be reduced, and maintenance costs can be saved. In terms of environmental protection, the ineffective allochroic silicagel is regenerated, so that environmental pollution can be reduced.

### BRIEF DESCRIPTION OF DRAWINGS

FIG. 1 is a system configuration diagram according to an embodiment of the disclosure.
FIG. 2 is a structure diagram of a data collection terminal according to an embodiment of the disclosure.
FIG. 3 is a structure diagram of a cloud server terminal according to an embodiment of the disclosure.
FIG. 4 is a front view of a connection portion between a respirator and a data collection terminal according to an embodiment of the disclosure.
FIG. 5 is a top view of a connection portion between a respirator and a data collection terminal of the disclosure.

In the drawings:
1- mounting flange,
2- moisture-absorbing barrel of respirator,
3- metal thin film filter,
4- mounting hole of respirator status data collection module and
5- embedded central processing module.

### DETAILED DESCRIPTION

The following further describes the technical solution of the disclosure in detail with reference to the accompanying drawings.

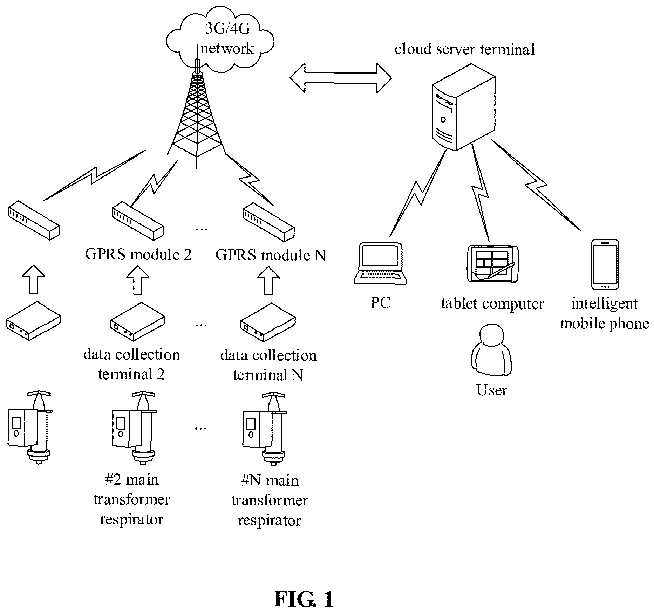

As shown in FIG. 1, an online monitoring and regeneration system for a power transformer respirator according to an embodiment of the disclosure includes: a power transformer respirator (such as a main transformer respirator shown in FIG. 1), a data collection terminal, a cloud server terminal and a monitoring terminal, the power transformer respirator is connected to the data collection terminal, and the data collection terminal, the cloud server terminal and the monitoring terminal are connected through a wireless network.

The data collection terminal is configured to collect operating status data of the power transformer respirator and image information including allochroic silicagel of the power transformer respirator, and send the operating status data and the image information to the cloud server terminal;
The cloud server terminal is configured to store the operation status data and the image information; and
the monitoring terminal is configured to communicate with the cloud server terminal, obtain the operation status data and the image information, and display the output of the operation status data and the image information.

The data collection terminal includes a respirator status data collection module configured to obtain operating status data of the power transformer respirator;
an image information collection module configured to obtain the image information including the allochroic silicagel of the power transformer respirator;
an embedded central processing module 5 configured to process the status data of the and the image information; and
a power management module configured to supply power to the respirator status data collection module, the image information collection module and the embedded central processing module 5.

The embedded central processing module includes an ARM processor, a data storage module, a human-computer interaction system, a data transmission module, a clock module, a sound and light alarm module and a heating control module.

In the embodiment of the disclosure, the power transformer respirator includes a mounting flange 1, a moisture-absorbing barrel 2 with the allochroic silicagel and a metal thin film filter 3. The mounting flange 1 connects an air tube of a transformer oil conservator and an air outlet of the power transformer respirator. The mounting flange 1 is connected to the moisture-absorbing barrel 2 through a connecting flange, the moisture-absorbing barrel 2 is connected to the metal thin film filter through bolts, and a bottom edge of the moisture- absorbing barrel 2 is in contact with the metal thin film filter 3 with an O-type sealing ring. In the embodiment of the disclosure, the power transformer respirator is mounted in a flange manner. The mounting flange 1 connects the air tube of the transformer oil conservator and the air outlet of the power transformer respirator. The mounting flange is connected to the moisture-absorbing barrel 2 through the connecting flange, the moisture absorption barrel 2 is connected to the metal thin film filter 3 through two long bolts, and the bottom edge of the moisture absorption barrel 2 is in contact with the metal thin film filter 3 with the O-type sealing ring. The metal thin film filter 3 replaces the oil cup in the conventional respirator to filter out the moisture and impurities in the air and avoid work of cleaning the oil cup; and the moisture-absorbing barrel containing the allochroic silicagelthe is used for drying the moisture.

In FIG. 1, the data collection terminal obtain the operation status data of the power transformer respirator connected thereto and the image information including the allochroic silicagel of the power transformer respirator, and uploads the obtained data to the cloud server terminal for storage through a GPRS module or a 3G / 4G wireless network. The monitoring terminal and the cloud server terminal exchange information through the network. The operating status of the power transformer respirator can be acquired in real time through a visual interface of the monitoring terminal, and remote online monitoring of the operating status of the power transformer respirator can be implemented. The monitoring terminal automatically controls execution of the corresponding operations according to the monitoring status of the transformer respirator to implement regeneration of the allochroic silicagel in the respirator.

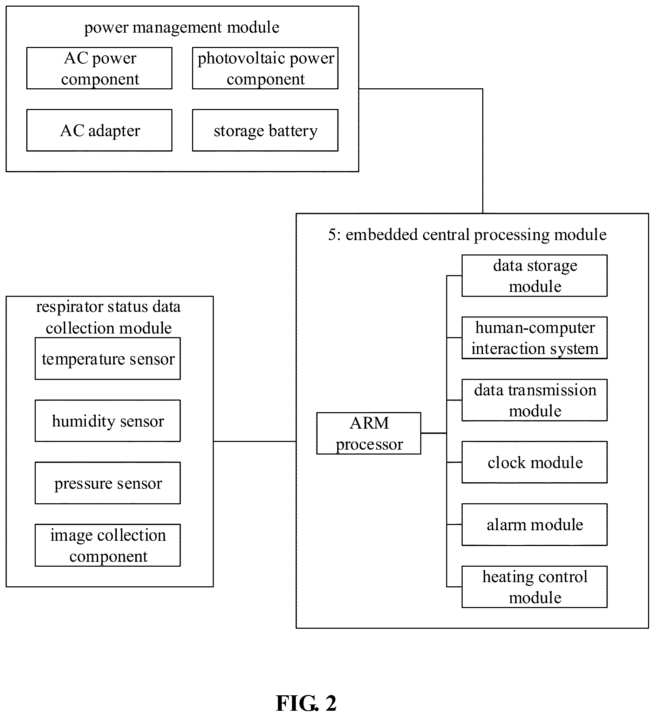

FIG. 2 is a structure schematic diagram of a data collection terminal according to an embodiment of the disclosure. As shown in FIG.2, the power management module may include an AC power component (the AC power is, for example, a 220V AC power), a photovoltaic power component and a storage battery. The power management module may further include an AC adapter. The AC power component can be connected to the storage battery through the AC adapter and then connected to the embedded central processing module 5, the photovoltaic power component is connected to the storage battery and then connected to the embedded central processing module 5, and the storage battery can be directly connected to the embedded central processing module 5. In the embodiment of the disclosure, a double-power supply mode of a solar cell and the AC power supply can be used. When there is a 220V AC power supply, the solar cell is smartly disconnected through a DC low voltage relay; and when the 220V AC power supply is disconnected, the solar cell is used as a power supply, the solar cell is connected to a solar panel through an intelligent solar charger. The charger charges the cell when the sun is full. The cell is automatically disconnected when being fully charged, and a charging current can reach 4A or more under sufficient sunlight, which fully meets circuit requirements.

The respirator status data collection module can include a temperature sensor (as an example, the temperature sensor may be an optical fiber distributed temperature sensor), a humidity sensor and a pressure sensor which are located at a connection between the mounting flange and the moisture-absorbing barrel and are connected to the embedded central processing module through a communication cable. The optical fiber distributed temperature sensor is used to collect multiple point temperature of the moisture-absorbing barrel of the power transformer respirator to achieve collection of an overall temperature in the moisture-absorbing barrel; the humidity sensor is used to continuously collect the air humidity in the respirator to know about water absorption conditions of a drying agent (the allochroic silicagel) in the respirator; and the pressure sensor is used to continuously collect pressure value in the respirator to monitor whether the respirator is internally unobstructed or not.

The respirator status data collection module further includes an image collection component. The image information collection module includes a camera configured to collect a color image of the allochroic silicagel in the moisture-absorbing barrel. The camera is mounted in the vicinity of the power transformer respirator to monitor color changes of the drying agent (the allochroic silicagel) in the respirator. As an example, a distance between the camera and the power transformer respirator may be about 2m to ensure obtaining a clear image of a color of the allochroic silicagel in the moisture-absorbing barrel of the power transformer respirator. The camera can transmit the image data collected by the camera to the embedded central processing module 5 in the form of data flow through wireless communication so as to continuously monitor of the color changes of the drying agent in the power transformer respirator.

In practical applications, the embedded central processing module 5 may include a processor (for example, an ARM processor) and the following modules connected to the processor: a data storage module, a human-computer interaction system, a data transmission module, a clock module, an alarm module and a heating control module. In practical applications, the processor may use a Cortex-A8 architecture-based ARM processor. The ARM processor is connected to the data storage module, the human-computer interaction system, the data transmission module, the clock module, the alarm module and the heating control module respectively.

The data storage module is configured to store the status data of the respirator and the image information. In practical applications, the data storage module may be divided into two parts of internal storage and external storage. The internal storage may be a Nand Flash with a capacity of 1GB, and is configured to store an operating system kernel, a log file system, a user application and the like, and may store collected data for at least one year by default. The external storage can be expanded by an extended memory card (for example, a TF card), and the capacity of the extended memory card can be selected according to actual site requirements, and the maximum of capacity can be up to 64G.

The human-computer interaction system includes a display component and an input component, and the input component may include a touch screen and/or a keyboard. As an example, the display component may be a 3.5-inch 64K true-color display screen for displaying various parameters of the system device and various kinds of collected data. The input component is for user operations such as viewing or setting parameters.

The data transmission module includes a wireless communication module and a data transmission interface, and the data transmission interface includes at least one of the following interface types: an RS485 interface, a USB interface and an RJ45 interface. Among them, the wireless communication module may use a GPRS module developed by an SIM900A chip, and establish a wireless communication link with a network side (for example, a base station and a core network) through the GPRS module to realize information interaction between the data collection terminal and the cloud server terminal, thereby realizing remote wireless transmission of the operation status data of the respirator and the image information and sending the collected data to the cloud server terminal.

The clock module uses a built-in real-time clock (RTC) to ensure the accuracy of a sampling period of status data such as the temperature and humidity of the respirator. The clock module also includes a power supply configured to supply power to the RTC. For example, the RTC can continue to work for about 20 years under the state of system power failure.

The alarm module is configured to perform an alarm when the processor determines that the power transformer respirator fails based on the obtained operating status data and image information of the power transformer respirator. The alarm module includes a buzzer and/or an indicator lamp, which can use a Light Emitting Diode (LED) indicator lamp. When the power transformer respirator fails (for example, the heating control module fails and pressure in the respirator is excessively high), the corresponding LED indicator lamp gives a red light alarm, and/or, the buzzer is triggered to given an alarm, so that operation staff of a substation can inspect and check.

The heating control module includes an allochroic silicagel heater, a metal thin film filter heater and a relay. The allochroic silicagel heater is located in the moisture-absorbing barrel, and the metal thin film filter heater is located in the metal thin film filter and close to the metal filter screen in the filter. The allochroic silicagel heater and the metal thin film filter heater are both connected to the embedded central processing module through a cable. The relay is located on a circuit board of the embedded central processing module. Startup or shutdown of the two heaters are controlled by pull-in or pull-off of contacts of the relay. When the air humidity in the power transformer respirator exceeds a programmed value, it indicates that the drying agent (i.e., the allochroic silicagel) temporarily loses the ability to absorb water. At this time, the heating control module controls the corresponding relay actions and starts the allochroic silicagel to heat the silicagel, so that the color changed silicagel is regenerated. When an ambient temperature is lower than the programmed value, the heating control module controls the corresponding relay actions and starts the metal thin film filter heater to heat the metal thin film filter to prevent the metal thin film filter from being condensed or frozen at a low temperature, thereby ensuring that the respirator maintains in a good working condition.

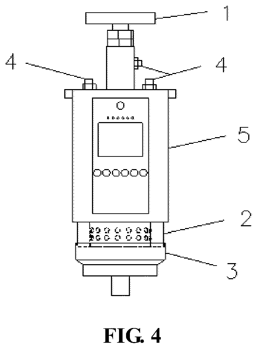

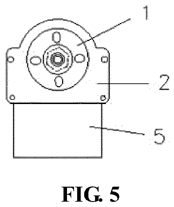

As shown in FIG. 4 and FIG. 5, a mounting hole 4 of the respirator status data collection module is provided in an upper part of the power transformer respirator, and the embedded central processing module 5 is mounted on a housing of the moisture-absorbing barrel 2 of the respirator through bolts. The respirator status data collection module may be arranged at the connection between the mounting flange and the moisture-absorbing barrel and is connected to the embedded central processing module 5 through a communication cable, and the embedded central processing module 5 is connected to the housing of the moisture-absorbing barrel 2 through bolts.

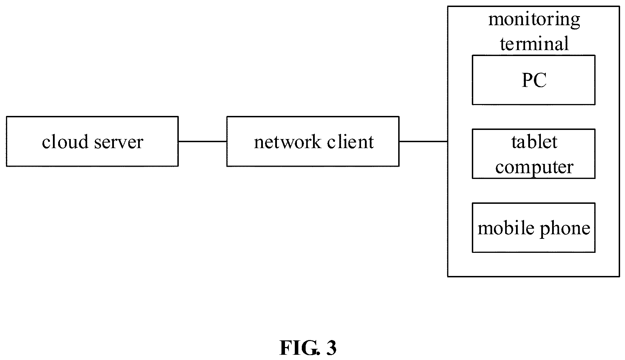

In an example, as shown in FIG. 3, the cloud server terminal may include a cloud server, a network client, a database and the like. The cloud server is configured to listen to the designated port, identify and accept connection requests sent by the data collection terminal and give corresponding service responses to implement operations for receiving, parsing, storing various kinds of data. The network client is configured to support real-time monitoring of the operating status of the power transformer respirator through the login of a terminal user, set status parameters of the power transformer respirator and control regeneration of the allochroic silicagel of the power transformer respirator. The database is configured to store and manage data and store the operating status and various parameters of the power transformer respirator(for example, the air humidity, temperature and pressure in the power transformer respirator and heating starting states, heating time and heating times of the respirators, and user information). The types of the monitoring terminal may include a PC, a tablet computer and an intelligent mobile phone. The monitoring terminal is configured to display related information of the power transformer respirator in real time to realize the function of online monitoring.

The monitoring terminal is configured to display the relevant data uploaded by the online monitoring and regeneration system for power transformer respirator in real time to realize the function of online monitoring. The monitoring terminal is further configured to log in to the network client of the cloud server terminal to set the parameters. For example, a set humidity value is suggested to be 20∼30%. If the set humidity value is too low, there is no substantial meaning. Instead, it will cause the device to start frequently. The set humidity value may be adjusted in a program. There may be two set temperature values. One is a control temperature for regeneration of the silicagel, and for example, may be set to 120 DEG C. If the temperature is too high, it may bring hidden dangers to personal safety of the user. The other set temperature value is a starting temperature of the metal thin film filter heater, and for example, is set to be 5 DEG C to prevent condensed water from being frozen to block an air hole in cold weather.

The embodiments of the disclosure have great effect and significance on the requirements of the smart grid monitoring, the intelligent operation and maintenance of the substation and environmental protection.

The above description is only the preferred embodiments of the disclosure and is not intended to limit the protection scope of the disclosure.

### INDUSTRIAL APPLICABILITY

The technical solutions of the embodiments of the disclosure apply embedded technology, optical fiber sensing technology, image collection technology, wireless communication technology and cloud server technology to status data collection, storage, transmission and remote monitoring of the power transformer respirator to realize online monitoring and regeneration of the power transformer respirator. On the other hand, the shutdown time of the transformer can be reduced, and the reliability of power supply reliability can be improved. In terms of safe operation, insulation performance of the transformer can be improved, and safe operation of equipment can be ensured. In terms of economical operation, a replacement frequency of the allochroic silicagel can be reduced, and maintenance costs can be saved. In terms of environmental protection, the ineffective allochroic silicagel is regenerated, so that environmental pollution can be reduced.

## Claims

1. An online monitoring and regeneration system for a power transformer respirator, comprising: a power transformer respirator, a data collection terminal, a cloud server terminal and a monitoring terminal, wherein the power transformer respirator is connected to the data collection terminal, the data collection terminal is connected to the cloud server terminal through a wireless network, and the cloud server terminal is connected to the monitoring terminal through the wireless network;
the data collection terminal is configured to obtain operating status data of the power transformer respirator and image information including allochroic silicagel of the power transformer respirator, and send the operating status data and the image information to the cloud server terminal;
the cloud server terminal is configured to store the operating status data and the image information; and
the monitoring terminal is configured to communicate with the cloud server terminal, and obtain and output the operating status data and the image information.

2. The online monitoring and regeneration system for the power transformer respirator according to claim 1, wherein the data collection terminal comprises:
a respirator status data collection module configured to obtain the operating status data of the power transformer respirator;
an image information collection module configured to obtain the image information including the silicagel of the power transformer respirator;
an embedded central processing module configured to process the operating status data of the power transformer respirator and the image information; and
a power management module configured to supply power to the respirator status data collection module, the image information collection module and the embedded central processing module.

3. The online monitoring and regeneration system for the power transformer respirator according to claim 1, wherein the power transformer respirator comprises a mounting flange, a moisture-absorbing barrel with the allochroic silicagel and a metal thin film filter; and
the mounting flange is connected to an air tube of an transformer oil conservator and an air outlet of the power transformer respirator, the mounting flange is connected to the moisture-absorbing barrel through a connecting flange, the moisture-absorbing barrel is connected to the metal thin film filter through bolts, and a bottom edge of the moisture-absorbing barrel is connected to the metal thin film filter with an O-type sealing ring.

4. The online monitoring and regeneration system for the power transformer respirator according to claim 3, wherein
the respirator status data collection module comprises a temperature sensor, a humidity sensor and a pressure sensor which are located at a connection of the mounting flange and the moisture-absorbing barrel and are connected to the embedded central processing module through a communication cable;
the image information collection module comprises a camera configured to collect a color image of the allochroic silicagel in the moisture-absorbing barrel, and the image information collection module is connected to the embedded central processing module through the wireless network to transmit image data collected by the camera to the embedded central processing module in the form of data flow; and
the embedded central processing module is connected to a housing of the moisture-absorbing barrel through bolts.

5. The online monitoring and regeneration system for the power transformer respirator according to claim 2, wherein the embedded central processing module comprises a processor and the following modules connected to the processor: a data storage module, a human-computer interaction system, a data transmission module, a clock module, an alarm module and a heating control module;
the data storage module is configured to store the respirator status data of and the image information;
the human-computer interaction system comprises a display component and an input component;
the data transmission module comprises a wireless communication module and a data transmission interface, and the data transmission interface comprises at least one of the following interface types: an RS485 interface, a Universal Serial Bus (USB) interface and an RJ45 interface;
the clock module uses a built-in Real Time Clock, abbreviated as RTC, and the clock module further comprises a power supply supplying power to the RTC; and
the alarm module is configured to give an alarm when the processor determines that the power transformer respirator fails based on the obtained operating status data of the power transformer respirator and image information, and the alarm module comprises a buzzer and/or an indicator lamp.

6. The online monitoring and regeneration system for the power transformer respirator according to claim 5, wherein the heating control module comprises an allochroic silicagel heater, a metal thin film filter heater and a relay;
the allochroic silicagel heater is located in the moisture-absorbing barrel, and the metal thin film filter heater is located in the metal thin film filter and close to a metal filter screen in the filter;
the allochroic silicagel heater and the metal thin film filter heater are both connected to the embedded central processing module through a cable, the relay is located on a circuit board of the embedded central processing module, and startup or shutdown of the two heaters is controlled respectively by pull-in or pull-out of contacts of the relay.

7. The online monitoring and regeneration system for the power transformer respirator according to claim 2, wherein the power management module comprises an Alternating Current power component, abbreviated as AC power component, a photovoltaic power component and a storage battery; and
when the power management module comprises the AC power component, the power management module further comprises an AC adapter, and the AC power component is connected to the AC adapter.

8. The online monitoring and regeneration system for the power transformer respirator according to claim 5, wherein the wireless communication module comprises a General Packet Radio Service module, abbreviated as GPRS module.

9. The online monitoring and regeneration system for the power transformer respirator according to claim 1, wherein the cloud server terminal comprises:
a cloud server configured to identify and accept connection requests sent by the data collection terminal and give service responses to receive, parse and store data;
a network client configured to monitor operating status of the power transformer respirator in real time after a user logins in, and set status parameters to control regeneration of the allochroic silicagel of the power transformer respirator; and
a database configured to store and manage the operating status parameters of the power transformer respirators and user information.

10. The online monitoring and regeneration system for the power transformer respirator according to claim 1, wherein types of the monitoring terminal comprise a Personal Computer, abbreviated as PC, a tablet computer and a mobile phone.
